(19) Europäisches Patentamt
European Patent Office
Office européen des brevets

(11) Veröffentlichungsnummer: **0 120 341**
**B1**

(12) # EUROPÄISCHE PATENTSCHRIFT

(45) Veröffentlichungstag der Patentschrift: **25.01.89**

(51) Int. Cl.[4]: **C 07 C 175/00**, C 07 D 317/72

(21) Anmeldenummer: **84102224.7**

(22) Anmeldetag: **02.03.84**

(54) **Verfahren zur Herstellung von Pentadienol-Derivaten.**

(30) Priorität: **25.03.83 CH 1646/83**

(43) Veröffentlichungstag der Anmeldung: **03.10.84 Patentblatt 84/40**

(45) Bekanntmachung des Hinweises auf die Patenterteilung: **25.01.89 Patentblatt 89/4**

(84) Benannte Vertragsstaaten: **CH DE FR GB IT LI**

(56) Entgegenhaltungen:
**EP-A-0 004 621**
**DE-A-2 032 906**
**DE-A-2 625 259**
**FR-A-2 467 837**

**Die Akte enthält technische Angaben, die nach dem Eingang der Anmeldung eingereicht wurden und die nicht in dieser Patentschrift enthalten sind.**

(73) Patentinhaber: **F. HOFFMANN- LA ROCHE & CO. Aktiengesellschaft, CH- 4002 Basel (CH)**

(72) Erfinder: **Lukàc, Teodor, Klusstrasse 32, CH- 4147 Aesch (CH)**
Erfinder: **Soukup, Milan, Dr., Blumenweg, CH- 4332 Stein (CH)**
Erfinder: **Widmer, Erich, Dr., Mittelweg 47, CH- 4142 Münchenstein (CH)**

(74) Vertreter: **Zimmermann, Hans, Dr., Grenzacherstrasse 124 Postfach 3255, CH- 4002 Basel (CH)**

**EP 0 120 341 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die vorliegende Erfindung betrifft ein Verfahren zur Herstellung von Zwischenprodukten in Carotinoidsynthesen, nämlich die Herstellung von 3-Methyl-2,4-pentadien-1-ol-Derivaten.

DE-A-2 625 259 beschreibt die katalytische Hydrierung von 5-(Trimethyl-3-oxocycloalk-1-enyl)-3-methyl-2-penten-4-in-1-ol-Verbindungen zu den entsprechenden 3-Methyl-2,4-pentadien-1-ol-Derivaten.

Aus DE-A-2 032 906 ist ferner bekannt, dass Acetylencarbinole wie 3-Methyl-5-(4-äthylendioxy-2,6,6-trimethyl-1-hydroxycyclohex-2-en-1-yl)-2-penten-4-in-1-ol mit Hilfe von Niederalkoxyniederalkenyloxy-alkalialuminium-hydriden selektiv zu Äthylencarbinolen hydriert werden können.

EP-A-4621 offenbart die Herstellung von 1-(5-Hydroxy-3-methyl-3-penten-1-inyl)-2,2,6-trimethyl-1,4-cyclohexandiol durch Alkinylierung eines geschützten 2,2,6-Trimethyl-1,4-cyclohexandions, Abspaltung der Schutzgruppe und Reduktion der Carbonylgruppe mit Niederalkoxy-alkalimetall-aluminiumhydrid. Das erhaltene Triol wird nach Veresterung der primären und sekundären Hydroxygruppen zum Cyclohexanderivat dehydratisiert.

Das erfindungsgemässe Verfahren ist dadurch gekennzeichnet, dass man in einem inerten organischen Lösungsmittel ein cyclisches Keton der allgemeinen Formel

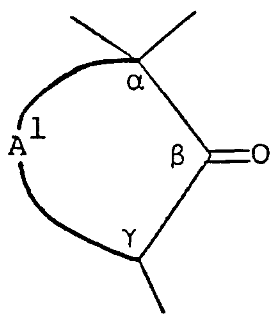

I

worin $A^1$ eine gegebenenfalls durch 1 oder 2 Äthergruppen substituierte Kette von 2 oder 3 Kohlenstoffatomen bezeichnet, deren Enden mit den Kohlenstoffatomen in α- und γ-Stellung verknüpft sind, mit einem Lithiumsalz der allgemeinen Formel

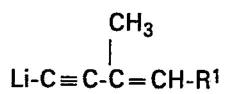

II

worin $R^1$ die Gruppe $-CHR^2R^3$ oder $-CH_2OLi$ darstellt,
$R^2$ Wasserstoff oder eine Äthergruppe bezeichnet und
$R^3$ ein Äthergruppe bedeutet,
umsetzt, die erhaltene Verbindung der allgemeinen Formel

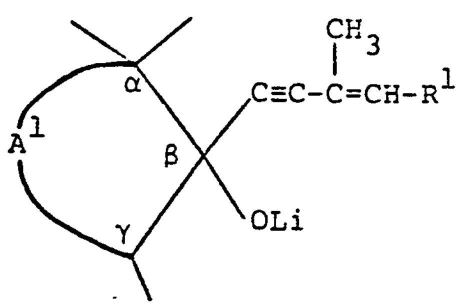

III

worin $A^1$ und $R^1$ die obigen Bedeutungen haben, mit einem Aluminiumhydrid der allgemeinen Formel

$$(C_nH_{2n+1}\text{-}O\text{-}C_mH_{2m}O)_xMeAlH_{4-x} \qquad \text{(IV)}$$

worin Me ein Alkalimetall, x eine Zahl von 1 bis 3 und m und n je eine Zahl von 1 bis 7 bezeichnen, reduziert und anschliessend zur Verbindung der allgemeinen Formel

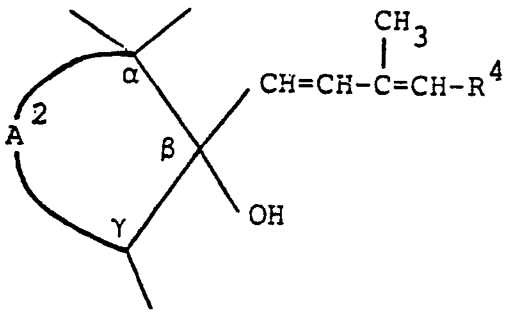

V

worin $R^4$ eine der Gruppen $-CH^2R^3$, $-CH_2OH$ oder -CHO darstellt, $R^2$ und $R^3$ die in Formel II gegebenen Bedeutungen haben, $A^2$ eine Kette von 2 oder 3 Kohlenstoffatomen bezeichnet, welche gegebenenfalls durch 1 oder 2 Hydroxy- oder Äthergruppen substituiert ist oder eine Oxogruppe aufweist, und die Enden der Kohlenstoffkette $A^2$ mit den Kohlenstoffatomen in α- und γ-Stellung verknüpft sind,

hydrolysiert und, gewünschtenfalls, eine erhaltene Verbindung der Formel V, worin Gruppe $A^2$ mit dem Kohlenstoffatom in γ-Stellung über eine einfache Kovalenzbindung verknüpft ist, zu einer Verbindung der allgemeinen Formel

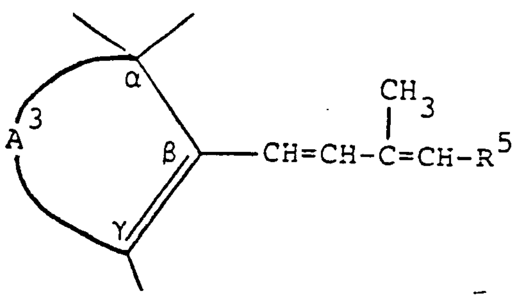

VI

worin $R^5$ die Gruppe $-CH_2OH$ oder -CHO darstellt, $A^3$ eine Kette von 2 oder 3 Kohlenstoffatomen bezeichnet, welche gegebenenfalls 1 oder 2 Hydroxygruppen oder eine Oxogruppe aufweist, und die Enden der Kohlenstoffkette $A^3$ mit den Kohlenstoffatomen in α- und γ-Stellung verknüpft sind,

dehydratisiert.

Die obige Gruppe $A^1$ umfasst definitionsgemäss die Gruppen $-CH_2-CH_2-$, $-CH_2-CH=$, $-CH=CH-$, $-CH_2-CH_2-CH_2-$, $-CH_2-CH_2-CH=$, $-CH_2-CH=CH-$, $-CH=CH-CH_2-$, und $-CH=CH-CH=$ und die davon abgeleiteten Gruppen, worin 1 oder 2 Wasserstoffatome durch Ätherfunktionen ersetzt sind. Die Verbindungen der Formel I weisen somit einen carbocyclischen, 5- oder 6-gliedrigen Ring auf. Die Bedeutung der Gruppen $A^2$ und $A^3$ ergibt sich sinngemäss, wenn man berücksichtigt, dass bei der Abspaltung der Äthergruppen die entsprechenden Hydroxyverbindungen bzw. im Falle geminaler Diäther (Ketale) die entsprechenden Carbonylverbindungen gebildet werden und Enole zu entsprechenden Ketonen umlagern können.

Der oben im Zusammenhang mit $A^1$, $A^2$ und $R^1$ vorkommende Ausdruck "Äthergruppe" umfasst alle als Schutzgruppen für Alkohole, Ketone und Aldehyde üblicherweise verwendbaren Ätherfunktionen. Beispiele solcher Gruppen sind Benzyloxy, Tetrahydropyranyloxy, $C_1$-$C_7$-Alkoxy (z. B. Methoxy, t-Butyloxy, Isobutyloxy), Silyloxygruppen (z. B. Trimethylsilyloxy), die Gruppen der allgemeinen Formel

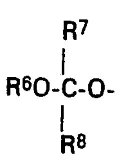

VII

worin $R^6$ $C_1$-$C_7$-Alkyl bedeutet und $R^7$ und $R^8$ Wasserstoff oder $C_1$-$C_7$-Alkyl bezeichnen,

(z. B. 1-Methoxy-1-methyläthoxy) und dergleichen. Im Falle geminaler und vicinaler Diäther können die beiden Ätherfunktionen auch zusammen eine cyclische Gruppe bilden. Solche Gruppen liegen beispielsweise dann vor, wenn ein Keton oder Aldehyd durch Ketalisierung bzw. Acetalisierung mit einem Diol, vorzugsweise mit Äthylenglykol oder Butan-2,3-diol, geschützt wird, wenn ein vicinales Diol durch Ketalisierung mit einem Keton, vorzugsweise mit Aceton, geschützt wird oder wenn ein enolisiertes α-Hydroxyketon durch Verätherung zum Dimer geschützt wird. Beispiele bevorzugter Äthergruppen sind Methoxy, Isobutyloxy, 1-Methoxy-1-methyläthoxy sowie im Falle geminaler Diäther auch Äthylendioxy und im Falle vicinaler Diäther auch die

Acetonidgruppe. Ferner sind im Falle der von enolisierten α-Hydroxyketonen (z. B. 3,4-Dihydroxy-2,5,5-trimethyl-2-cyclopenten-1-on) abgeleiteten Verbindungen der Formel I auch die durch Verätherung zum Dimer gebildeten vicinalen Diäther bevorzugt.

Der Ausdruck "Alkalimetall" bedeutet im Rahmen der vorliegenden Erfindung Lithium, Natrium und Kalium, insbesondere Lithium und Natrium.

Das erfindungsgemässe Verfahren bietet eine elegante Methode zur Herstellung der Verbindungen der Formeln V und VI in hoher Ausbeute. Da die Alkinylierung und die anschliessende Reduktion und Hydrolyse sowie gegebenenfalls auch die Dehydratisierung als Eintopfreaktion durchgeführt werden können, erübrigt sich eine zeitraubende und kostspielige Aufarbeitung der Zwischenprodukte. Da ferner die intermediären Salze der Formel III überraschend schnell und problemlos mit den Verbindungen der Formel IV komplexieren, erfolgt die Reduktion schon bei sehr milden Bedingungen praktisch augenblicklich und mit vergleichsweise geringen Mengen an Reduktionsmittel.

Vorzugsweise bedeutet $A^1$ in obiger Formel I eine der Gruppen $-CH_2-CH=$, $-CH_2-CH_2-CH_2-$, $-CH_2-CH_2-CH=$ oder $-CH=CH-CH=$ oder eine davon abgeleitete Gruppe, worin 1 oder 2 Wasserstoffatome durch Ätherfunktionen ersetzt sind. Besonders bevorzugte Ausgangsmaterialien in dem erfindungsgemässen Verfahren sind diejenigen Verbindungen der Formel I, worin $A^1$ eine der Gruppen $-CHR-CR'=$, $-CH_2-CR=$, $-CH_2-CH_2-CH_2-$, $-CH_2-CHR-CH_2-$, $-CH_2-CRR'-CH_2-$, $-CH_2-CH_2-CR=$, $-CH_2-CHR-CR'=$ oder $-CH_2-CRR'-CH=$ bezeichnet und R und R' Äthergruppen bedeuten. Beispiele besonders bevorzugter Ausgangsmaterialien der Formel I sind 4-(1-Methoxy-1-methyläthoxy)-2,2,6-trimethylcyclohexan-1-on, 7,9,9-Trimethyl-1,4-dioxaspiro[4.5]dec-6-en-8-on, 7,7,9-Trimethyl-1,4-dioxaspiro-[4.5]decan-8-on, 2,2,4,6,6-Pentamethyl-7,7a-dihydro-2H,6H,1,3-benzodioxol-5-on, 2,2,6-Trimethyl-5-isobutyloxy-5-cyclohexen-1-on, 2,2,6-Trimethylcyclohexanon und cis-1,4a,5,8a-Tetra-hydro-1,1,3,5,5,7-hexamethyldicyclopenta-b-dioxin-2,6-dion.

Das erfindungsgemässe 3- bzw. 4-Stufen-Verfahren wird in einem inerten organischen Lösungsmittel durchgeführt, beispielsweise in einem Äther oder einem gesättigten oder aromatischen Kohlenwasserstoff, wie Tetrahydrofuran, Dioxan, Diäthyläther, Petroläther, Hexan, Benzol, Toluol, Xylol und dergleichen. Bevorzugtes Lösungsmittel ist Tetrahydrofuran.

Die Alkinylierung eines Ketons der Formel I mit einem Lithiumsalz der Formel II kann in an sich bekannter Weise erfolgen. Temperatur und Druck sind nicht kritisch. Im allgemeinen wird jedoch bei Atmosphärendruck und Raumtemperatur oder tieferer Temperatur, vorzugsweise bei etwa -30°C bis Raumtemperatur, gearbeitet. Die Alkinylierung kann mit äquimolaren Mengen an Verbindungen der Formeln I und II durchgeführt werden. Im allgemeinen wird jedoch ein geringer Überschuss an Verbindung der Formel II bevorzugt.

Die Reduktion des Lithiumalkoholates der Formel III kann unter den üblichen Bedingungen durchgeführt werden, die bei Reduktionen mit Aluminiumhydriden der Formel IV normalerweise angewendet werden. Temperatur und Druck sind nicht kritisch. Da die vorliegende Reduktion jedoch schon bei tiefen Temperaturen rasch abläuft, wird vorzugsweise bei etwa -50°C bis Raumtemperatur gearbeitet. Zweckmässigerweise wird die Reduktion so durchgeführt, dass das Reduktionsmittel nach beendeter Alkinylierungsreaktion direkt zum erhaltenen Reaktionsgemisch zugegeben wird. Die Dreifachbindung in Formel III wird im allgemeinen fast ausschliesslich zur trans-Doppelbindung reduziert. Me in Formel IV bedeutet vorzugsweise Lithium oder Natrium. Ferner sind diejenigen Reduktionsmittel der Formel IV bevorzugt, worin x für die Zahl 2 steht. m und n bezeichnen vorzugsweise je eine Zahl von 1 bis 3. Besonders bevorzugtes Reduktionsmittel ist Natrium-bis(2-methoxyäthoxy)aluminiumhydrid. Im allgemeinen werden etwa äquivalente Mengen an Verbindungen der Formeln III und IV oder, vorzugsweise, ein geringer Überschuss an Verbindung der Formel IV eingesetzt.

Die anschliessende Hydrolyse des intermediären Aluminiumkomplexes und, sofern Gruppe $A^2$ in Formel V mit dem Kohlenstoffatom in γ-Stellung über eine einfache Kovalenzbindung verknüpft ist, gewünschtenfalls die Dehydratisierung zu einer Verbindung der Formel VI kann nach den an sich bekannten Methoden der Hydrolyse und Dehydratisierung mit üblichen Reagenzien durchgeführt werden. Die optimalen Bedingungen können je nach Substrat, Schutzgruppen und gewünschtem Produkt variieren, können aber von Fall zu Fall leicht ermittelt werden.

Die Hydrolyse des intermediären Aluminiumkomplexes kann beispielsweise mit Wasser, mit wässriger Ammoniumchlorid-, Acetatpuffer- oder Phosphatpuffer-Lösung, mit Laugen, wie Natronlauge oder Kalilauge, mit organischen oder anorganischen Säuren, wie p-Toluolsulfonsäure, Schwefelsäure und dergleichen erfolgen. Die Hydrolyse kann gewünschtenfalls so durchgeführt werden, dass gleichzeitig auch die gegebenenfalls vorhandenen Äthergruppen hydrolysiert werden. Die Bedingungen, unter denen die hier verwendeten Schutzgruppen erhalten bleiben bzw. abgespalten werden, sind dem Fachmann grundsätslich bekannt. Im allgemeinen kann jedoch gesagt werden, dass die Schutzgruppen unter neutralen oder basischen Bedingungen und gelegentlich auch unter schwach sauren Bedingungen erhalten bleiben, während sie unter sauren, insbesondere unter stark sauren Bedingungen abgespalten werden. Temperatur und Druck sind nicht kritisch. Im allgemeinen wird jedoch bei Atmosphärendruck und Raumtemperatur oder tieferer Temperatur, vorzugsweise bei etwa 0°C bis Raumtemperatur gearbeitet.

Die Dehydratisierung einer Verbindung der Formel V, worin Gruppe $A^2$ mit dem Kohlenstoffatom in γ-Stellung über eine einfache Kovalenzbindung verknüpft ist, zu einer Verbindung der Formel VI kann mit üblichen, in der Literatur bekannten Dehydratisierungsreagenzien, beispielsweise auch mit starken organischen oder anorganischen Säuren, wie p-Toluolsulfonsäure, Schwefelsäure und dergleichen, durchgeführt werden. Die optimalen Bedingungen können je nach Substrat variieren. Im allgemeinen sind jedoch Atmosphärendruck und eine Temperatur von Raumtemperatur bis Rückflusstemperatur bevorzugt. Eine

bevorzugte Methode zur Herstellung der Verbindungen der Formel VI besteht darin, dass der nach der Reduktion erhaltene Aluminiumkomplex unter den für die Dehydratisierung angegebenen Bedingungen mit einer starken organischen oder anorganischen Säure behandelt wird, wodurch in einem Schritt Hydrolyse des Komplexes, Abspaltung gegebenenfalls vorhandener Schutzgruppen und Dehydratisierung erreicht werden kann. Anderseits kann es bei einigen schwer dehydratisierbaren Verbindungen der Formel V von Vorteil sein, diese zuerst in leichter dehydratisierbare Derivate überzuführen. Beispielsweise wird die Dehydratisierung von 5-(1,4-Dihydroxy-2,2,6-trimethylcyclohexyl)-3-methyl-2,4-pentadien-1-ol erleichtert, wenn dieses zuerst zum Diacetat verestert wird.

Die Ausgangsmaterialien der Formeln I und II sind bekannte oder Analoge bekannter Verbindungen und können in bekannter oder an sich bekannter Weise hergestellt werden.

Die Acetylenide der Formel II können mit Vorteil in situ hergestellt werden aus dem entsprechenden Acetylenderivat durch Umsetzung mit einer geeigneten Lithiumverbindung, beispielsweise Phenyllithium oder einem Alkyllithium, wie Methyllithium, Butyllithium und dergleichen. Zweckmässigerweise wird diese Umsetzung bei Atmosphärendruck und etwa -30°C bis Raumtemperatur im gleichen Lösungsmittel durchgeführt, wie das anschliessende erfindungsgemässe Verfahren.

Die Erfindung betrifft ferner alle neuen Verbindungen, Mischungen, Verfahren und Verwendungen wie hierin beschrieben.

Das erfindungsgemässe Verfahren wird durch die folgenden Beispiele weiter veranschaulicht.

**Beispiel 1**

In einem Sulfierkolben mit Rührer, Thermometer und Tropftrichter mit Druckausgleich wurden unter Argonbegasung 14,5 g 5-(1-Methoxy-1-methyläthoxy)-3-methyl-3E-penten-1-in (96%-ig, 83 mMol) in 90 ml Tetrahydrofuran gelöst und mit einem Aceton/Trockeneis-Bad auf -15°C gekühlt. Bei dieser Temperatur wurde unter gutem Kühlen innert 30 Minuten eine Lösung von 60 ml einer 1,4M Lösung von Butyllithium (80 mMol) in Hexan zugesetzt und dann das Gemisch noch 30 Minuten bei 0°C unter Argon nachgerührt. Anschliessend wurde unter Rühren, Argonbegasung und Kühlen bei -10°C innert 30 Minuten eine Lösung von 4(R)-(1-Methoxy-1-methyl-äthoxy)-2,2,6(R)-trimethylcyclohexan-1-on in Tetrahydrofuran zugetropft, dann das Kühlbad entfernt und das Reaktionsgemisch vorsichtig auf Raumtemperatur erwärmt und 1 Stunde nachgerührt.

Das Reaktionsgemisch wurde auf -10°C gekühlt. Anschliessend wurden 25 ml einer 70 %-igen (Gew.-%) Lösung von Natrium-bis(1-methoxyäthoxy)aluminiumhydrid (90 mMol) in Toluol mit 75 ml Tetrahydrofuran versetzt und die erhaltene Lösung innert 15 Minuten zum Reaktionsgemisch zugesetzt. Das Reaktionsgemisch wurde noch weitere 15 Minuten bei -10°C nachgerührt.

Anschliessend wurde das Reaktionsgemisch bei -10°C und unter kräftigem Rühren innert 5 Minuten mit 50 ml Äthanol versetzt, dann innert 10 Minuten unter gutem Kühlen tropfenweise mit 200 ml 3N Schwefelsäure versetzt und schliesslich noch 1 Stunde bei 0°C nachgerührt. Drei Scheidetrichter $S_1$-$S_3$ wurden mit je 150 ml Essigsäureäthylester beschickt. Dann wurden der Reihe nach und unter guter Durchmischung zuerst das Reaktionsgemisch und dann dreimal je 150 ml halbgesättigte wässrige Natriumhydrogencarbonat-Lösung durch die Scheidetrichter $S_1$-$S_3$ geschickt. Die wässrigen Phasen wurden verworfen. Die organischen Phasen wurden vereinigt und über Natriumsulfat getrocknet. Das Trocknungsmittel wurde abfiltriert, mit 50 ml Essigsäureäthylester nachgewaschen und das Filtrat im Rotationsverdampfer am Wasserstrahlvakuum bei 40°C bis zur Gewichtskonstanz getrocknet, wobei als Rückstand 24,0 g semikristalline gelbe Masse erhalten wurde. Dieses Rohprodukt wurde auf dem Dampfbad unter Rückfluss in 90 ml Essigsäureäthylester klar gelöst, dann unter Rühren langsam auf Raumtemperatur abgekühlt und über Nacht bei -20°C gelagert. Das auskristallisierte Produkt wurde abgenutscht, zweimal mit je 10 ml kaltem (-20°C) Essigsäureäthylester gewaschen und im Trockenschrank am Wasserstrahlvakuum bei 40°C bis zur Gewichtskonstanz getrocknet. Hierbei wurden 9,6 g farblose Kristalle mit Smp. 156-161°C, $\alpha_D$ = -56,6° erhalten. Die Mutterlauge wurde im Rotationsverdampfer am Wasserstrahlvakuum eingedampft und dann am Hochvakuum weitgehend von 3-Methyl-2-penten-4-in-1-ol befreit. Das zurückbleibende Öl (9,4 g) wurde in analoger Weise zur 1. Kristallisation aus 30 ml Essigsäureäthylester kristallisiert, wobei weitere 0,6 g Produkt in Form farbloser Kristalle mit Smp. 153 - 159°C erhalten wurden. Gesamtausbeute: 10,2 g (67 % bezogen auf 4(R)-Hydroxy-2,2,6(R)-trimethylcyclohexan-1-on) gemäss Dünnschichtchromatographie reines 5-(1(R,S),4(R)-Dihydroxy-2,2,6(R)-trimethylcyclohexyl]-3-methyl-2E,4E-pentadien-1-ol (1R/1S-Verhältnis 95 : 5).

Die oben verwendete Lösung von 4(R)-(1-Methoxy-1-methyl-äthoxy)-2,2,6(R)-trimethylcyclohexan-1-on in Tetrahydrofuran wurde wie folgt hergestellt:

In einem Sulfierkolben mit Rührer, Thermometer und Tropftrichter mit Druckausgleich wurde eine Lösung von 9,4 g 4(R)-Hydroxy-2,2,6(R)-trimethylcyclohexan-1-on (60 mMol) in 50 ml Tetrahydrofuran vorgelegt und unter Argonbegasung und Rühren auf 15°C abgekühlt. Nach Zusatz von 20 mg p-Toluolsulfonsäuremonohydrat wurden innert 45 Minuten bei 15 - 20°C 8,7 g Isopropenylmethyläther (120 mMol) zugetropft (Kühlung mit Methanol/Eis-Bad). Die erhaltene Lösung von 4(R)-(1-Methoxy-1-methyläthoxy)-2,2,6(R)-trimethylcyclohexan-1-on wurde mit 0,2 ml Triäthylamin versetzt, auf -10°C gekühlt und bis zur Verwendung unter Argon gelagert.

**Beispiel 2**

In einem Sulfierkolben mit Rührer, Thermometer und Tropftrichter mit Druckausgleich wurden unter Argonbegasung 24,1 g 5,5-Dimethoxy-3-methyl-3Z-penten-1-in (166 mMol) in 150 ml absolutem Tetrahydrofuran gelöst und die Lösung mit einem Aceton/Trockeneis-Bad auf -15°C gekühlt. Bei dieser Temperatur wurden unter gutem Kühlen innert 30 Minuten 120 ml einer 1,4M Lösung von Butyllithium (160 mMol) in Hexan zugesetzt und die entstandene Lithiumsalzlösung noch 30 Minuten bei 0°C nachgerührt. Anschliessend wurde unter Rühren, Argonbegasung und Kühlen bei -10°C innert 15 Minuten eine Lösung von 23,8 g 7,9,9-Trimethyl-1,4-dioxaspiro[4.5]dec-6-en-8-on (120 mMol) in 100 ml Tetrahydrofuran zum Reaktionsgemisch zugetropft. Dann wurde das Kühlbad entfernt und das Reaktionsgemisch vorsichting auf Raumtemperatur erwärmt und 2 Stunden nachgerührt.

Das Reaktionsgemisch wurde auf -45°C gekühlt. Anschliessend wurden 50 ml einer 70 %-igen (Gew.-%) Lösung von Natrium-bis(2-methoxyäthoxy)aluminiumhydrid (180 mMol) in Toluol mit 140 ml Tetrahydrofuran versetzt und die erhaltene Lösung innert 15 Minuten zum Reaktionsgemisch zugetropft.

Anschliessend wurde das Reaktionsgemisch bei -45°C und unter wirksamem Rühren innert 10 Minuten tropfenweise mit 100 ml Äthanol versetzt, dann bei -10°C bis 0°C mit 300 ml halbgesättigter wässriger Ammoniumchlorid-Lösung versetzt und noch 1 Stunde bei 0°C gerührt. Drei Scheidetrichter $S_1$-$S_3$ wurden mit je 250 ml Essigsäureäthylester beschickt. Dann wurden der Reihe nach und unter guter Durchmischung zunächst das Reaktionsgemisch und dann dreimal je 200 ml halbgesättigte wässrige Kochsalzlösung durch die Scheidetrichter $S_1$-$S_3$ geschickt. Die wässrigen Phasen wurden verworfen. Die organischen Phasen wurden vereinigt und über Natriumsulfat getrocknet. Das Trocknungsmittel wurde abfiltriert und mit 80 ml Essigsäureäthylester nachgewaschen. Das Filtrat wurde im Rotationsverdampfer am Wasserstrahlvakuum bei 40°C eingedampft und am Hochvakuum weitgehend vom 5,5-Dimethoxy-3-methyl-3Z-penten-1-in befreit (3 Stunden bei 50°C/0,15 mm Hg). Als Rückstand wurden 43,2 g rohes 8-(5,5-Dimethoxy-3-methyl-1E,3Z-pentadienyl)-8-hydroxy-7,9,9-trimethyl-1,4-dioxaspiro[4.5]dec-6-en in Form eines gelblichen Öles erhalten.

Das erhaltene Öl wurde in einem Kolben mit Thermometer, Magnetrührer und Argonbegasung in 80 ml Aceton und 50 ml entionisiertem Wasser gelöst. Nach Zusatz von 0,2 g p-Toluolsulfonsäure wurde das Reaktionsgemisch 45 Minuten bei Raumtemperatur gerührt. Drei Scheidetrichter $S_1$-$S_3$ wurden mit je 200 ml Essigsäureäthylester beschickt. Dann wurden der Reihe nach und unter guter Durchmischung zunächst das Reaktionsgemisch und dann dreimal je 80 ml gesättigte wässrige Natriumhydrogencarbonat-Lösung durch die Scheidetrichter $S_1$-$S_3$ geschickt. Die wässrigen Phasen wunden verworfen. Die organischen Phasen wurden vereinigt und über Natriumsulfat getrocknet. Das Trocknungsmittel wurde abfiltriert und mit 60 ml Essigsäureäthylester nachgewaschen. Das Filtrat wurde im Rotationsverdampfer am Wasserstrahlvakuum bei 40°C bis zur Gewichtskonstanz eingedampft, wobei 35,8 g gelbliches Öl zurückblieben. Das erhaltene Öl wurde auf dem Dampfbad unter Rückfluss in 100 ml Diisopropyläther und 10 ml Essigsäureäthylester gelöst, dann unter Rühren langsam auf Raumtemperatur abgekühlt und über Nacht bei -20°C stehengelassen. Das auskristallisierte Produkt wurde abgenutscht, auf der Nutsche zweimal mit je 20 ml kaltem (-20°C) Diisopropyläther gewaschen und im Trockenschrank am Wasserstrahlvakuum bei 40°C bis zur Gewichtskonstanz getrocknet. Hierbei wurden 20,9 g (70,1 %) 5-(1-Hydroxy-4-oxo-2,6,6-trimethyl-2-cyclohexenyl)-3-methyl-2Z,4E-pentadienal in Form farbloser Kristalle mit Smp. 111-113°C erhalten.

**Beispiel 3**

In einem Sulfierkolben mit Rührer, Thermometer und Tropftrichter mit Druckausgleich wurden unter Argonbegasung 14,7 g 5-(1-Methoxy-1-methyläthoxy)-3-methyl-3Z-penten-1-in (83 mMol) in 90 ml absolutem Tetrahydrofuran gelöst und die Lösung mit einem Aceton/Trockeneis-Bad auf -15°C gekühlt. Bei dieser Temperatur wurden innert 10 Minuten unter gutem Kühlen 60 ml einer 1,4M Lösung von Butyllithium (80 mMol) in Hexan zum Reaktionsgemisch zugesetzt und die entstandene Lithiumsalzlösung noch 30 Minuten bei 0°C unter Argon nachgerührt. Anschliessend wurde innert 8 Minuten unter Rühren, Argonbegasung und Kühlen bei -10°C eine Lösung von 11,9 g 7,9,9-Trimethyl-1,4-dioxaspiro[4.5]-dec-6-en-8-on (60 mMol) in 60 ml Tetrahydrofuran zum Reaktionsgemisch zugetropft. Dann wurde das Kühlbad entfernt und das Reaktionsgemisch vorsichtig auf Raumtemperatur erwärmt und 1,5 Stunden nachgerührt.

Das Reaktionsgemisch wurde auf -25°C gekühlt. Anschliessend wurden 14 ml einer 70 %-igen (Gew.-%) Lösung von Natrium-bis(2-methoxyäthoxy)aluminiumhydrid (50,2 mMol) in Toluol mit 80 ml Tetrahydrofuran versetzt und die erhaltene Lösung innert 10 Minuten zum Reaktionsgemisch zugetropft. Dann wurde das Kühlbad entfernt und das Reaktionsgemisch vorsichtig auf -10°C erwärmt und 30 Minuten nachgerührt. Anschliessend wurde das Reaktionsgemisch bei -20°C innert 10 Minuten tropfenweise mit 30 ml Äthanol versetzt, dann bei ca. -10°C bis 15°C mit 200 ml 3N Schwefelsäure versetzt und noch 2 Stunden bei 15°C bis Raumtemperatur nachgerührt. Drei Scheidetrichter $S_1$-$S_3$ wurden mit je 150 ml Essigsäureäthylester beschickt. Dann wurden der Reihe nach und unter guter Durchmischung zunächst das Reaktionsgemisch und dann dreimal je 150 ml gesättigte Natriumhydrogencarbonat-Lösung durch die Scheidetrichter $S_1$-$S_3$ geschickt. Die wässrigen Phasen wurden verworfen. Die organischen Phasen wurden vereinigt und über Natriumsulfat getrocknet. Das Trocknungsmittel wurde abfiltriert und mit 80 ml Essigsäureäthylester nachgewaschen. Das

Filtrat wurde im Rotationsverdampfer am Wasserstrahlvakuum bei 40°C eingedampft und schliesslich am Hochvakuum von 3-Methyl-2Z-penten-4-in-1-ol weitgehend befreit (2,5 Stunden bei 55°C/0,1 mm Hg). Als Rückstand wurden 19,5 g rohes 5-(1-Hydroxy-4-oxo-2,6,6-trimethyl-2-cyclohexenyl)-3-methyl-2Z,4E-pentadien-1-ol in Form einer gelblichen kristallinen Masse erhalten. Das erhaltene Rohprodukt wurde auf dem Dampfbad unter Rückfluss in 25 ml Essigsäureäthylester und 25 ml Diisopropyläther gelöst und die Lösung unter Rühren langsam auf Raumtemperatur abgekühlt und über Nacht bei -20°C stehengelassen. Das auskristallisierte Produkt wurde abgenutscht, auf der Nutsche zweimal mit je 15 ml kaltem (-20°C) Diisopropyläther gewaschen und dann im Trockenschrank am Wasserstrahlvakuum bei 40°C bis zur Gewichtskonstanz getrocknet. Hierbei wurden 9,3 g (62 %) 5-(1-Hydroxy,4-oxo-2,6,6-trimethyl-2-cyclohexenyl)-3-methyl-2Z,4E-pentadien-1-ol in Form farbloser Kristalle mit Smp. 127 - 129°C erhalten.

**Beispiel 4**

In einem Sulfierkolben mit Rührer, Thermometer und Tropftrichter mit Druckausgleich wurden unter Argonbegasung 24,0 g 3-Methyl-2Z-penten-4-in-1-ol (249,6 mMol) in 150 ml absolutem Tetrahydrofuran gelöst und die Lösung mit einem Aceton/Trockeneis-Bad auf -20°C gekühlt. Bei dieser Temperatur wurden unter gutem Kühlen innert 20 Minuten 308 ml einer 1,56M Lösung von Butyllithium (481,2 mMol), in Hexan zugetropft und das entstandene Dilithiumsalz (beige Suspension) noch 25 Minuten bei -10°C unter Argon nachgerührt. Anschliessend wurde unter Rühren, Argonbegasung und Kühlen bei -5°C innert 10 Minuten eine Lösung von 23,8 g 7,9,9-Trimethyl-1,4-dioxaspiro[4.5]dec-6-en-8-on (120 mMol) in 100 ml Tetrahydrofuran zum Reaktionsgemisch zugetropft. Dann wurden 2 g Tetrabutylammoniumbromid (6,2 mMol) zum Reaktionsgemisch gegeben, das Kühlbad entfernt und das Gemisch vorsichtig auf Raumtemperatur erwärmt und 3,75 Stunden nachgerührt.

Das Reaktionsgemisch wurde auf -25°C gekühlt. Anschliessend wurden 60 ml einer 70 %-igen (Gew.-%) Lösung von Natrium-bis(2-methoxyäthoxy)aluminiumhydrid (215 mMol) in Toluil mit 80 ml Tetrahydrofuran versetzt und die erhaltene Lösung innert 10 Minuten zum Reaktionsgemisch zugetropft. Dann wurde das Kühlbad entfernt und das Reaktionsgemisch vorsichtig auf Raumtemperatur erwärmt (wobei sich die Suspension löste und die Farbe nach weinrot wechselte) und 1 Stunde nachgerührt.

Anschliessend wurde das Reaktionsgemisch bei -20°C innert 10 Minuten tropfenweise mit 100 ml Äthanol versetzt (wobei die Farbe von weinrot nach hellgelb wechselte), dann bei ca. -10°C bis 12°C mit 500 ml 3N Schwefelsäure versetzt und noch 15 Minuten bei 12°C bis Raumtemperatur nachgerührt. Drei Scheidetrichter $S_1$-$S_3$ wurden mit je 400 ml Essigsäureäthylester beschickt. Dann wurden der Reihe nach und unter guter Durchmischung zunächst das Reaktionsgemisch und dann dreimal je 250 ml gesättigte Natriumhydrogencarbonatlösung durch die Scheidetrichter $S_1$-$S_3$ geschickt. Die wässrigen Phasen wurden verworfen. Die organischen Extrakte wurden vereinigt und über Natriumsulfat getrocknet. Das Trocknungsmittel wurde abfiltriert und mit 80 ml Essigsäureäthylester nachgewaschen. Das Filtrat wurde im Rotationsverdampfer am Wasserstrahlvakuum bei 40°C eingedampft. Der erhaltene Rückstand (44,1 g gelbliche Kristalle) wurde dreimal mit je 300 ml Pentan auf dem Dampfbad unter Rückfluss digeriert. Vor dem Abdekantieren des Pentans wurde jeweils mit einem Eis/Wasser-Bad gekühlt. Einengen der gesammelten Pentanextrakte im Rotationsverdampfer am Wasserstrahlvakuum bei 40°C ergab 11,6 g gelbliches Öl. Der beim Digerieren verbleibende kristalline Rückstand wurde ebenfalls im Rotationsverdampfer am Wasserstrahlvakuum bei 40°C bis zur Gewichtskonstanz getrocknet, wobei 29,3 g gelbliches kristallines Rohprodukt von 5-(1-Hydroxy-4-oxo-2,6,6-trimethyl-2-cyclohexenyl)-3-methyl-2Z,4E-pentadien-1-ol erhalten wurden. Dieses Rohprodukt wurde auf dem Dampfbad unter Rückfluss in 70 ml Essigsäureäthylester gelöst und die warme Lösung unter Rühren innert 10 Minuten tropfenweise mit 35 ml Hexan versetzt. Dann wurde die Lösung innert 3 Stunden auf Raumtemperatur abgekühlt und über Nacht bei -20°C stehengelassen. Das auskristallisierte Produkt wurde abgenutscht, auf der Nutsche dreimal mit je 20 ml kaltem (-20°C) Hexan gewaschen und nachher im Trockenschrank am Wasserstrahlvakuum bei 40°C bis zur Gewichtskonstanz getrocknet. Hierbei wurden 24,0 g (80,0 %) farblose Kristalle mit Smp. 126-128°C erhalten. Eindampfen der Mutterlauge im Rotationsverdampfer am Wasserstrahlvakuum ergab 5,3 g gelbliches Öl, aus welchem durch Chromatographie und Kristallisation der Produktfraktionen aus Essigsäureäthylester/Hexan weitere 0,8 g (2,6 %) farblose Kristalle mit Smp. 127 - 128°C isoliert werden konnten. Gesamtausbeute: 24,8 g (82,6 %) 5-(1-Hydroxy-4-oxo-2,6,6-trimethyl-2-cyclohexenyl)-3-methyl-2Z,4E-pentadien-1-ol.

**Beispiel 5**

In einem Sulfierkolben mit Rührer, Thermometer und Tropftrichter mit Druckausgleich wurden unter Argonbegasung 14,5 g 5-(1-Methoxy-1-methyläthoxy)-3-methyl-3E-penten-1-in (96 %-ig, 83 mMol) in 90 ml Tetrahydrofuran gelöst und mit einem Aceton/Trockeneis-Bad auf -15°C gekühlt. Bei dieser Temperatur wurde unter gutem Kühlen innert 15 Minuten 60 ml einer 1,4M Lösung von Butyllithium (80 mMol) in Hexan zugesetzt und die entstandene Lithiumsalzlösung noch 30 Minuten bei 0°C nachgerührt. Anschliessend wurde unter

Rühren, Argonbegasung und Kühlen bei -10°C innert 10 Minuten eine Lösung von 12,6 g 2,6,6-Trimethyl-3-isobutoxycyclohex-2-en-1-on (60 mMol) in 50 ml Tetrahydrofuran zum Reaktionsgemisch zugetropft. Dann wurde das Kühlbad entfernt und das Reaktionsgemisch vorsichtig auf Raumtemperatur erwärmt und 1 Stunde nachgerührt.

Das Reaktionsgemisch wurde auf -10°C gekühlt. Anschliessend wurden 34 ml einer 70 %-igen (Gew.-%) Lösung von Natrium-bis(2-methoxyäthoxy)aluminiumhydrid (120 mMol) in Toluol mit 60 ml Tetrahydrofuran versetzt und die erhaltene Lösung innert 10 Minuten zum Reaktionsgemisch zugetropft. Dann wurde das Kühlbad entfernt und das Reaktionsgemisch vorsichtig auf Raumtemperatur erwärmt und 2 Stunden nachgerührt.

Anschliessend wurde das Reaktionsgemisch bei -10°C innert 5 Minuten tropfenweise mit 30 ml Äthanol versetzt, dann bei ca. -10°C bis +5°C mit 200 ml 3N Schwefelsaure versetzt und noch 2 Stunden bei ca. 5°C nachgerührt. Drei Scheidetrichter $S_1$-$S_3$ wurden mit je 150 ml Essigsäureäthylester beschickt. Dann wurden der Reihe nach und unter guter Durchmischung zunächst das Reaktionsgemisch und dann dreimal je 150 ml gesättigte Natriumhydrogencarbonat-Lösung durch die Scheidetrichter $S_1$-$S_3$ geschickt. Die wässrigen Phasen wurden verworfen. Die organischen Phasen wurden vereinigt und über Natriumsulfat getrocknet. Das Trocknungsmittel wurde abfiltriert und mit 80 ml Essigsäureäthylester nachgewaschen. Das Filtrat wurde im Rotationsverdampfer am Wasserstrahlvakuum bei 40°C eingedampft und schliesslich im Hochvakuum von 3-Methyl-2E-penten-4-in-1-ol weitgehend befreit (2 Stunden bei 50°C/0,1 mmHg). Als Rückstand wurden 17,8 g rohes 3-[5-Hydroxy-3-methyl-1E,3E-pentadienyl]-2,4,4-trimethyl-2-cyclohexen-1-on in Form eines gelblichen Öles erhalten. Das erhaltene Öl wurde über 430 g Kieselgel mit n-Hexan/-Äther 2 : 1 chromatographiert. Die produkthaltigen Fraktionen wurden vereinigt und am Rotationsverdampfer bei 40°C am Wasserstrahlvakuum eingeengt. Nach Trocknen im Hochvakuum bei Raumtemperatur erhielt man 10,2 g (72,5 % bezogen auf 2,6,6-Trimethyl-3-isobutoxycyclohex-2-en-1-on) gemäss Dünnschichtchromatographie reines 3-[5-Hydroxy-3-methyl-1E,3E-pentadienyl]-2,4,4-trimethyl-2-cyclohexen-1-on.

**Beispiel 6**

In einem Sulfierkolben mit Rührer, Thermometer und Tropftrichter mit Druckausgleich wurden unter Argonbegasung 24,0 g 3-Methyl-2E-penten-4-in-1-ol (249,6 mMol) in 150 ml absolutem Tetrahydrofuran gelöst und die Lösung mit einem Aceton/Trockeneis-Bad auf -20°C gekühlt. Bei dieser Temperatur wurden unter gutem Kühlen innert 15 Minuten 308 ml einer 1,56M Lösung von Butyllithium (481,2 mMol) in Hexan zugetropft und das entstandene Dilithiumsalz (beige Suspension) noch 15 Minuten bei -10°C unter Argon nachgerührt. Anschliessend wurden unter Rühren, Argonbegasung und Kühlen bei -20°C innert 10 Minuten eine Lösung von 25,2 g 7,7a-Dihydro-2,2,4,6,6-pentamethyl-1,3-benzodioxol-5(6H)-on (120 mMol) in 150 ml Tetrahydrofuran zum Reaktionsgemisch zugetropft. Dann wurden 2 g Tetrabutylammoniumbromid (6,2 mMol) zum Reaktionsgemisch gegeben, das Kühlbad entfernt und das Gemisch vorsichtig auf Raumtemperatur erwärmt und 3 Stunden nachgerührt.

Das Reaktionsgemisch wurde auf -30°C gekühlt. Anschliessend wurden 60 ml einer 70 %-igen Lösung von Natrium-bis-(2-methoxyäthoxy)aluminiumhydrid (215 mMol) in Toluol mit 110 ml Tetrahydrofuran versetzt und die erhaltene Lösung innert 10 Minuten zum Reaktionsgemisch zugetropft. Dann wurde das Kühlbad entfernt und das Reaktionsgemisch vorsichtig auf Raumtemperatur erwärmt und 1 Stunde nachgerührt.

Anschliessend wurde das Reaktionsgemisch bei -20°C innert 15 Minuten tropfenweise mit 100 ml Äthanol versetzt, dann bei ca. -10°C bis 15°C mit 500 ml 3N Schwefelsäure versetzt und noch 30 Minuten bei 15°C bis Raumtemperatur nachgerührt. Drei Scheidetrichter $S_1$-$S_3$ wurden mit je 400 ml Essigsäureäthylester beschickt. Dann wurden der Reihe nach und unter guter Durchmischung zunächst das Reaktionsgemisch und dann dreimal je 250 ml gesättigte Natriumhydrogencarbonatlösung durch die Scheidetrichter $S_1$-$S_3$ geschickt. Die wässrigen Phasen wurden verworfen. Die organischen Extrakte wurden vereinigt und über Natriumsulfat getrocknet. Das Trocknungsmittel wurde abfiltriert und mit 80 ml Essigsäureäthylester nachgewaschen. Das Filtrat wurde im Rotationsverdampfer am Wasserstrahlvakuum bei 40°C eingedampft. Der erhaltene Rückstand (44,8 g gelbliches Öl) wurde an Kieselgel mit Diäthyläther/Hexan (Vol. 2 : 1) chromatographiert. Aus den produkthaltigen Fraktionen konnten 22,5 g (75,0 %) 6-Hydroxy-3-(5-hydroxy-3-methyl-1E,3E-pentadienyl)-2,4,4-trimethyl-2-cyclohexen-1-on als farblose Kristalle mit Smp. 85 - 86°C isoliert werden.

**Beispiel 7**

In einem Sulfierkolben mit Rührer, Thermometer und Tropftrichter mit Druckausgleich wurden unter Argonbegasung 24,0 g 3-Methyl-2Z-penten-4-in-1-ol (249,6 mMol) in 150 ml absolutem Tetrahydrofuran gelöst und die Lösung mit einem Aceton/Trockeneis-Bad auf -20°C gekühlt. Bei dieser Temperatur wurden unter gutem Kühlen innert 35 Minuten 308 ml einer 1,56M Lösung von Butyllithium (481,2 mMol) in Hexan zugetropft und das entstandene Dilithiumsalz (beige Suspension) noch 20 Minuten bei -10°C unter Argon nachgerührt. Anschliessend wurde unter Rühren, Argonbegasung und Kühlen bei -15°C innert 10 Minuten eine Lösung von

4(R)-(1-Methoxy-1-methyläthoxy)-2,2,6(R)-trimethyl-cyclohexan-1-on in absolutem Tetrahydrofuran zum Reaktionsgemisch zugetropft. Dann wurden 2 g Tetrabutylammoniumbromid (6,2 mMol) zum Reaktionsgemisch gegeben, das Kühlbad entfernt und das Gemisch vorsichtig auf Raumtemperatur erwärmt und 3 Stunden nachgerührt.

Das Reaktionsgemisch wurde auf -40°C gekühlt. Anschliessend wurden 60 ml einer 70 %-igen Lösung von Natrium-bis-(2-methoxyäthoxy)aluminiumhydrid (215 mMol) in Toluol mit 110 ml Tetrahydrofuran versetzt und die erhaltene Lösung innert 15 Minuten zum Reaktionsgemisch zugetropft. Dann wurde das Kühlbad entfernt und das Reaktionsgemisch vorsichtig auf +5°C erwärmt und 1 Stunde nachgerührt.

Anschliessend wurde das Reaktionsgemisch bei -30°C innert 15 Minuten tropfenweise mit 100 ml Äthanol, dann bei -15°C bis +5°C mit 300 ml halbgesättigter wässriger Ammoniumchlorid-Lösung versetzt und noch 1 Stunde bei ca. 5°C gerührt. Drei Scheidetrichter $S_2$-$S_3$ wurden mit je 300 ml Essigsäureäthylester beschickt. Dann wurden der Reihe nach und unter guter Durchmischung zunächst das Reaktionsgemisch und dann dreimal je 200 ml halbgesättigte wässrige Kochsalzlösung durch die Scheidetrichter $S_1$-$S_3$ geschickt. Die wässrigen Phasen wurden verworfen. Die organischen Phasen wurden vereinigt und über Natriumsulfat getrocknet. Das Trocknungsmittel wurde abfiltriert und mit 80 ml Essigsäureäthylester nachgewaschen. Das Filtrat wurde im Rotationsverdampfer am Wasserstrahlvakuum bei 40°C eingedampft. Als Rückstand wurden 51,3 g eines gelblichen Öls erhalten.

Das erhaltene Öl wurde in einem Kolben mit Thermometer, Magnetrührer und Argonbegasung in 200 ml Tetrahydrofuran und 10 ml entionisiertem Wasser gelöst. Nach Zusatz von 1,0 g Pyridinium-(toluol-4-sulfonat) (4 mMol) wurde das Reaktionsgemisch 1 Stunde bei Raumtemperatur gerührt und anschliessend auf 300 ml gesättigte Natriumhydrogencarbonat-Lösung in einen Scheidetrichter $S_1$ gegossen. Scheidetrichter $S_2$-$S_3$ wurden mit je 200 ml Essigsäureäthylester beschickt. Nun wurde die wässrige Phase aus $S_1$ unter intensiver Durchmischung durch die Scheidetrichter $S_2$-$S_3$ geschickt und anschliessend verworfen. Die vereinigten organischen Phasen aus $S_1$-$S_3$ wurden mit Natriumsulfat getrocknet. Das Trocknungsmittel wurde abfiltriert und mit 80 ml Essigsäureäthylester nachgewaschen. Das Filtrat wurde im Rotationsverdampfer am Wasserstrahlvakuum bei 40°C eingedampft. Der erhaltene Rückstand (40,3 g gelbliches Öl) wurde an Kieselgel mit Diäthyläther/Hexan (Vol. 3 : 1) chromatographiert. Aus den produkthaltigen Fraktionen konnten 27,3 g (89,5 %) 5-[1(R,S),4(R)-Dihydroxy-2,2,6(R)-trimethylcyclohexyl]-3-methyl-2Z,4E-pentadien-1-ol als Gemisch der beiden 1R/1S-Isomeren isoliert werden.

Die oben verwendete Lösung von 4(R)-(1-Methoxy-1-methyläthoxy)-2,2,6(R)-trimethylcyclohexan-1-on in Tetrahydrofuran wurde wie folgt hergestellt:

In einem Sulfierkolben mit Rührer, Thermometer und Tropftrichter mit Druckausgleich wurde eine Lösung von 18,75 g 4(R)-Hydroxy-2,2,6(R)-trimethylcyclohexan-1-on (120 mMol) in 100 ml Tetrahydrofuran vorgelegt und unter Argonbegasung und Rühren auf 15°C abgekühlt. Nach Zusatz von 100 mg Pyridinium-(toluol-4-sulfonat) wurden innert 30 Minuten bei 15 - 25°C 17,4 g Isopropenylmethyläther (240 mMol) zugetropft. Die erhaltene Lösung von 4(R)-(1-Methoxy-1-methyläthoxy)-2,2,6(R)-trimethylcyclohexan-1-on wurde auf -10°C gekühlt und bis Verwendung unter Argon gelagert.

**Beispiel 8**

In einem Sulfierkolben mit Rührer, Thermometer und Tropftrichter wurden unter Argonbegasung 56,4 g 5-(1-Methoxy-1-methyläthoxy)-3-methyl-3E-penten-1-in (97 %-ig, 325 mMol) in 300 ml absolutem Hexan gelöst und die Lösung mit einem Aceton/Trockeneis-Bad auf 10°C gekühlt. Bei dieser Temperatur wurden unter gutem Rühren innert 15 Minuten 192,3 ml einer 1,56M Lösung von Butyllithium (300 mMol) in Hexan zugetropft. Das Reaktionsgemisch wurde noch 1 Stunde bei 10°C gerührt und dann auf Raumtemperatur erwärmt. Anschliessend wurde innert 15 Minuten eine Lösung von 52,7 g 7,7a-Dihydro-2,2,4,6,6-pentamethyl-1,3-benzodioxol-5(6H)-on (250 mMol) in 120 ml Hexan zum Reaktionsgemisch zugetropft und das erhaltene Gemisch noch 3 Stunden bei Raumtemperatur gerührt.

Das Reaktionsgemisch wurde auf -40°C gekühlt. Anschliessend wurden 107,1 ml einer 70 %-igen Lösung von Natrium-bis(2-methoxyäthoxy)aluminiumhydrid (375 mMol) in Toluol innert 10 Minuten zum Reaktionsgemisch zugesetzt. Dann wurde das Reaktionsgemisch langsam auf 0°C erwärmt, 2 Stunden bei dieser Temperatur gehalten und anschliessend bei Raumtemperatur 16 Stunden nachgerührt.

Anschliessend wurde das Reaktionsgemisch bei -20°C innert 20 Minuten mit 250 ml Äthanol, dann bei -20°C bis +10°C mit 500 ml halbgesättigter Ammoniumchlorid-Lösung versetzt und gut gerührt. Drei Scheidetrichter $S_1$-$S_3$ wurden mit je 1 l Essigsäureäthylester beschickt. Dann wurden der Reihe nach und unter guter Durchmischung zunächst das Reaktionsgemisch und dann einmal 500 ml halbgesättigte Ammoniumchlorid-Lösung und zweimal je 1 l halbgesättigte Kochsalzlösung durch die Scheidetrichter $S_1$-$S_3$ geschickt. Die wässrigen Phasen wurden verworfen. Die organischen Phasen wurden vereinigt, über Natriumsulfat getrocknet und filtriert. Das Filtrat wurde im Rotationsverdampfer am Wasserstrahlvakuum bei 40°C eingeengt und am Hochvakuum bei 50°C 2 Stunden getrocknet. Der Rückstand (100,9 g) wurde in 1 l Tetrahydrofuran und 100 ml Wasser gelöst und mit 2,5 g p-Toluolsulfonsäure versetzt. Die Lösung wurde bei Raumtemperatur unter Argonbegasung 30 Minuten gerührt und dann in einem Scheidetrichter $S_1$ auf 500 ml Essigsäureäthylester gegossen. Scheidetrichter $S_2$ und $S_3$ wurden mit je 500 ml Essigsäureäthylester beschickt. Dann wurden unter

guter Durchmischung zweimal je 750 ml gesättigte Natriumhydrogencarbonat-Lösung durch die Scheidetrichter $S_1$-$S_3$ geschickt. Die wässrigen Phasen wurden verworfen. Die organischen Phasen wurden vereinigt, über Natriumsulfat getrocknet und filtriert. Das Filtrat wurde im Rotationsverdampfer am Wasserstrahlvakuum bei 40°C eingeengt und schliesslich am Hochvakuum bei 50°C 5 Stunden getrocknet. Der erhaltene Rückstand (69,9 g, Reinheit 78 %, Ausbeute 86 %) wurde an Kieselgel mit Diäthyläther/Hexan (Vol. 2: 1) chromatographiert. Aus den produkthaltigen Fraktionen wurden 48,2 g (77 %) 6-Hydroxy-3-(5-hydroxy-3-methyl-1E,3E-pentadienyl)-2,4,4-trimethyl-2-cyclohexen-1-on als farblose Kristalle mit Smp. 85 - 86°C erhalten.

**Patentansprüche**

1. Verfahren zur Herstellung von 3-Methyl-2,4-pentadien-1-ol-Derivaten, dadurch gekennzeichnet, dass man in einem inerten organischen Lösungsmittel ein cyclisches Keton der allgemeinen Formel

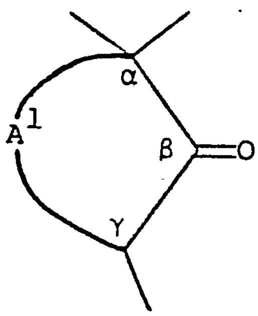

I

worin $A^1$ eine gegebenenfalls durch 1 oder 2 Äthergruppen substituierte Kette von 2 oder 3 Kohlenstoffatomen bezeichnet, deren Enden mit den Kohlenstoffatomen in α- und γ-Stellung verknüpft sind, mit einem Lithiumsalz der allgemeinen Formel

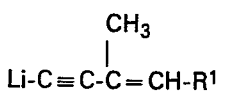

II

worin $R^1$ die Gruppen $-CHR^2R^3$ oder $-CH_2OLi$ darstellt,
$R^2$ Wasserstoff oder eine Äthergruppe bezeichnet und
$R^3$ eine Äthergruppe bedeutet,
umsetzt, die erhaltene Verbindung der allgemeinen Formel

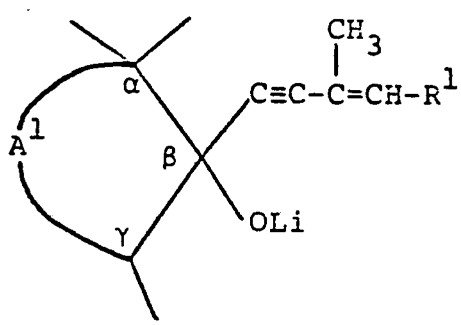

III

worin $A^1$ und $R^1$ die obigen Bedeutungen haben, mit einem Aluminiumhydrid der allgemeinen Formel

$$(C_nH_{2n+1}\text{-}O\text{-}C_mH_{2m}O)_xMeAlH_{4-x} \qquad IV$$

worin Me ein Alkalimetall, x eine Zahl von 1 bis 3 und m und n je eine Zahl von 1 bis 7 bezeichnen, reduziert und anschliessend zur Verbindung der allgemeinen Formel

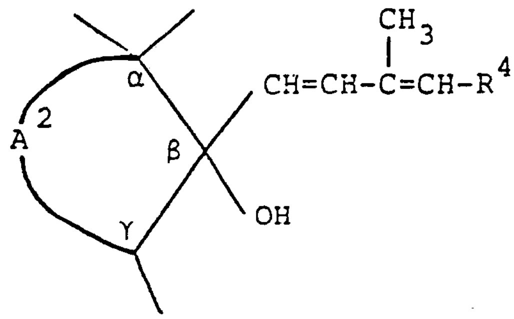

V

worin $R^4$ eine der Gruppen $-CH^2R^2$, $-CH_2OH$ oder -CHO darstellt, $R^2$ und $R^3$ die in Formel II gegebenen Bedeutungen haben, $A^2$ eine Kette von 2 oder 3 Kohlenstoffatomen bezeichnet, welche gegebenenfalls durch 1 oder 2 Hydroxy- oder Äthergruppen substituiert ist oder eine Oxogruppe aufweist, und die Enden der Kohlenstoffkette $A^2$ mit den Kohlenstoffatomen in α- und γ-Stellung verknüpft sind,
hydrolysiert und, gewünschtenfalls, eine erhaltene Verbindung der Formel V, worin Gruppe $A^2$ mit dem Kohlenstoffatom in γ-Stellung über eine einfache Kovalenzbindung verknüpft ist, zu einer Verbindung der allgemeinen Formel

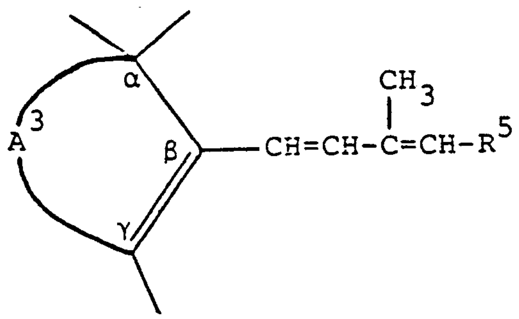

VI

worin $R^5$ die Gruppe $-CH_2OH$ oder -CHO darstellt, $A^3$ eine Kette von 2 oder 3 Kohlenstoffatomen bezeichnet, welche gegebenenfalls 1 oder 2 Hydroxygruppen oder eine Oxogruppe aufweist, und die Enden der Kohlenstoffkette $A^3$ mit den Kohlenstoffatomen in α- und γ-Stellung verknüpft sind,
dehydratisiert.

2. Verfahren nach Anspruch 1, dadurch gekennzeichnet dass man eine Verbindung der Formel I umsetzt worin $A^1$ eine der Gruppen $-CH_2-CH=$, $-CH_2-CH_2-CH_2-$, $-CH_2-CH_2-CH=$ oder $-CH=CH-CH=$ bedeutet oder eine davon abgeleitete Gruppe, worin 1 oder 2 Wasserstoffatome durch Äthergruppen ersetzt sind.

3. Verfahren nach Anspruch 2, dadurch gekennzeichnet, dass man eine Verbindung der Formel I umsetzt, worin $A^1$ eine der Gruppen $-CHR-CR'=$, $-CH_2-CR=$, $-CH_2-CH_2-CH_2-$, $-CH_2-CHR-CH_2-$, $-CH_2-CRR'-CH_2-$, $-CH_2-CH_2-CR=$, $-CH_2-CHR-CR'=$ oder $-CH_2-CRR'-CH=$ bezeichnet und R und R' Äthergruppen bedeuten.

4. Verfahren nach einem der Ansprüche 1 bis 3, dadurch gekennzeichnet, dass man eine Verbindung der Formel I umsetzt, worin die in $A^1$ gegebenenfalls vorhandenen Äthergruppen Methoxy, Isobutyloxy oder 1-Methoxy-1-methyläthoxy bedeuten oder zwei geminale Äthergruppen zusammen auch Äthylendioxy bedeuten oder zwei vicinale Äthergruppen zusammen auch die Acetonidgruppe bedeuten oder im Falle geschützter enolisierter α-Hydroxyketone die vicinalen Äthergruppen auch durch Verätherung zum Dimer gebildet sind.

5. Verfahren nach einem der Ansprüche 1 bis 4, dadurch gekennzeichnet, dass man die Umsetzung mit einer Verbindung der Formel II durchführt, worin gegebenenfalls in $R^1$ vorhandene Äthergruppen Methoxy, Isobutyloxy oder 1-Methoxy-1-methyläthoxy oder zwei Äthergruppen zusammen auch Äthylendioxy bedeuten.

6. Verfahren nach Anspruch 5, dadurch gekennzeichnet, dass man die Umsetzung mit der Verbindung der Formel II durchführt, worin $R^1$ die Gruppe $-CH_2OLi$ darstellt.

7. Verfahren nach einem der Ansprüche 1 bis 6, dadurch gekennzeichnet, dass man die Reduktion mit einer Verbindung der Formel IV durchführt, worin Me Lithium oder Natrium und x die Zahl 2 bedeuten.

8. Verfahren nach Anspruch 7, dadurch gekennzeichnet, dass man die Reduktion mit Natrium-bis(2-methoxyäthoxy)aluminiumhydrid durchführt.

9. Verfahren nach einem der Ansprüche 1 bis 8, dadurch gekennzeichnet, dass man die Umsetzung in einem Äther oder einem gesättigten oder aromatischen Kohlenwasserstoff durchführt.

10. Verfahren nach Anspruch 9, dadurch gekennzeichnet, dass man die Umsetzung in Tetrahydrofuran durchführt.

## Claims

1. A process for the manufacture of 3-methyl-2,4-pentadien-1-ol derivatives, characterized by reacting a cyclic ketone of the general formula

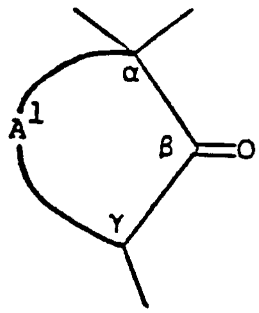

I

wherein $A^1$ denotes a chain of 2 or 3 carbon atoms which is optionally substituted by 1 or 2 ether groups and the ends of which are linked with the carbon atoms in the α- and γ-position,
with a lithium salt of the general formula

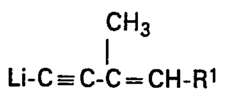

II

wherein $R^1$ represents the groups $-CHR^2R^3$ or
$-CH_2-OLi$,
$R^2$ denotes hydrogen or an ether group and
$R^3$ signifies an ether group,
in an inert organic solvent, reducing the resulting compound of the general formula

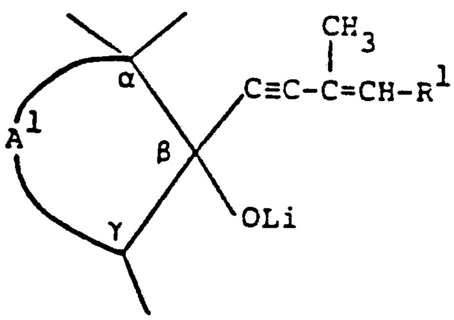

III

wherein $A^1$ and $R^1$ have the above significances, with an aluminium hydride of the general formula

$(C_nH_{2n+1}\text{-}O\text{-}C_mH_{2m}O)_xMeAlH_{4-x}$ IV

wherein Me denotes an alkali metal, x denotes a number of 1 to 3 and m and n each denote a number of 1 to 7,
and subsequently hydrolyzing to a compound of the general formula

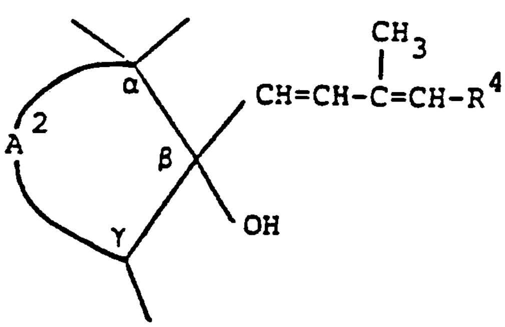

V

wherein $R^4$ represents one of the groups $-CHR^2R^3$, $-CH_2OH$ or -CHO, $R^2$ and $R^3$ have the significances given in formula II, $A^2$ denotes a chain of 2 or 3 carbon atoms, which is optionally substituted by 1 or 2 hydroxy or ether groups or has an oxo group, and the ends of the carbon chain $A^2$ are linked with the carbon atoms in the α- and γ-position,

and, if desired, dehydrating a resulting compound of formula V in which group $A^2$ is linked with the carbon atom in the γ-position via a single covalent bond to a compound of the general formula

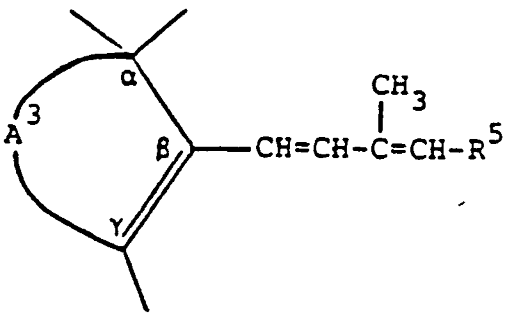

VI

wherein $R^5$ represents the group $-CH_2OH$ or -CHO, $A^3$ denotes a chain of 2 or 3 carbon atoms, which optionally has 1 or 2 hydroxy groups or an oxo group, and the ends of the carbon chain $A^3$ are linked with the carbon atoms in the α- and γ-position.

2. A process according to claim 1, characterized in that a compound of formula I in which $A^1$ signifies one of the groups $-CH_2-CH=$, $-CH_2-CH_2-CH_2-$, $-CH_2-CH_2-CH=$ or $-CH=CH-CH=$ or a group derived therefrom in which 1 or 2 hydrogen atoms is/are replaced by ether groups is reacted.

3. A process according to claim 2, characterized in that a compound of formula I in which $A^1$ denotes one of the groups $-CHR-CR'=$, $-CH_2-CR=$, $-CH_2-CH_2-CH_2-$, $-CH_2-CHR-CH_2-$, $-CH_2-CRR'-CH_2-$, $-CH_2-CH_2-CR=$, $-CH_2-CHR-CR'=$ or $-CH_2-CRR'-CH=$ and R and R' signify ether groups is reacted.

4. A process according to any one of claims 1 to 3, characterized in that there is reacted a compound of formula I in which the ether groups optionally present in $A^1$ signify methoxy, isobutyloxy, or 1-methoxy-1-methylethoxy or two geminal ether groups together also signify ethylenedioxy or two vicinal ether groups together also signify the acetonide group or in the case of protected enolized α-hydroxyketones the vicinal ether groups are also formed by etherification to the dimer.

5. A process according to any one of claims 1 to 4, characterized in that the reaction is carried out with a compound of formula II in which ether groups optionally present in $R^1$ signify methoxy, isobutyloxy or 1-methoxy-1-methylethoxy or two ether groups together also signify ethylenedioxy.

6. A process according to claim 5, characterized in that the reaction is carried out with the compound of formula II in which $R^1$ represents the group $-CH_2OLi$.

7. A process according to any one of claims 1 to 6, characterized in that the reduction is carried out with a compound of formula IV in which Me signifies lithium or sodium and x signifies the number 2.

8. A process according to claim 7, characterized in that the reduction is carried out with sodium bis(2-methoxyethoxy)aluminium hydride.

9. A process according to any one of claims 1 to 8, characterized in that the reaction is carried out in an ether or a saturated or aromatic hydrocarbon.

10. A process according to claim 9, characterized in that the reaction is carried out in tetrahydrofuran.

**Revendications**

1. Procédé de préparation de dérivés du 3-méthyl-2,4-pentadiène-1-ol caractérisé en ce que l'on fait réagir dans un solvant organique inerte une cétone cyclique de formule générale

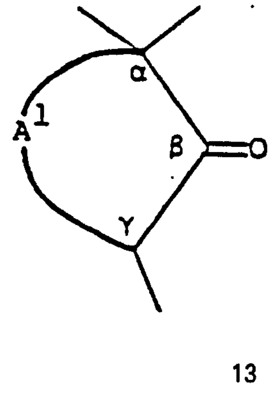

I

dans laquelle $A^1$ représente une chaîne à deux ou trois atomes de carbone éventuellement substituée par un ou deux groupes éther et dont les extrémités sont reliées aux atomes de carbone en positions alpha et gamma, avec un sel de lithium de formule générale

$$\mathrm{Li{-}C{\equiv}C{-}\underset{}{\overset{\overset{CH_3}{|}}{C}}{=}CH{-}R^1} \qquad \text{II}$$

dans laquelle $R^1$ represente les groupes $-CHR^2R^3$ ou $-CH_2OLi$,
$R^2$ représente l'hydrogène ou un groupe ether, et
$R^3$ représente un groupe éther,
ce qui donne un composé de formule générale

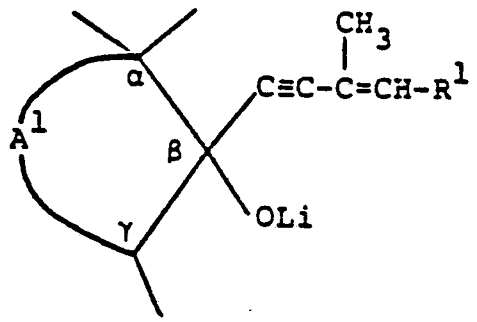

III

dans laquelle $A^1$ et $R^1$ ont les significations indiquées ci-dessus, qu'on réduit à l'aide d'un hydrure d'aluminium de formule générale

$$(C_nH_{2n+1}\text{-}O\text{-}C_mH_{2m}O)_xMeAlH_{4-x} \qquad \text{IV}$$

dans laquelle Me représente un métal alcalin, x est un nombre allant de 1 à 3 et m et n représentent chacun un nombre allant de 1 à 7,
puis on hydrolyse en le composé de formule générale

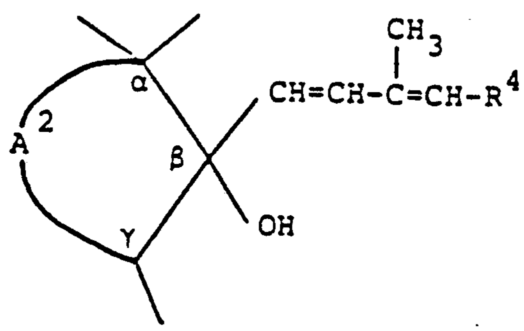

V

dans laquelle $R^4$ représente l'un des groupes $-CHR^2R^3$, $-CH_2OH$ ou $-CHO$, $R^2$ et $R^3$ ont les significations indiquées en référence à la formule II, $A^2$ représente une chaîne à deux ou trois atomes de carbone éventuellement substituée par un ou deux groupes hydroxy ou éther ou contenant un groupe oxo, et les extrémités de la chaîne carbonée $A^2$ sont reliées aux atomes de carbone dans les positions alpha et gamma,

et si on le désire, on convertit un composé obtenu répondant à la formule V dans laquelle le groupe $A^2$ est relié avec l'atome de carbone en position gamma par une liaison covalente simple, par déshydratation, en un composé de formule générale

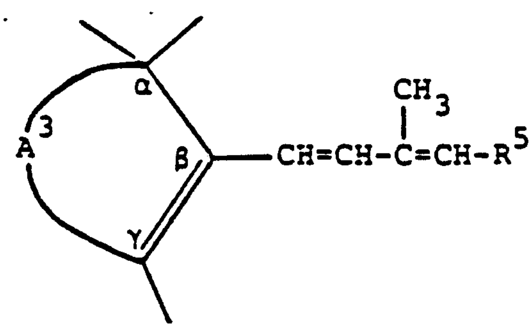

VI

dans laquelle $R^5$ représente le groupe $-CH_2OH$ ou le groupe -CHO, $A^3$ représente une chaîne à deux ou trois atomes de carbone contenant le cas échéant un ou deux groupes hydroxy ou un groupe oxo, et les extrémités de la chaîne carbonée $A^3$ sont reliées aux atomes de carbone dans les positions alpha et gamma.

2. Procédé selon la revendication 1, caractérisé en ce que l'on convertit un composé de formule I dans laquelle $A^1$ représente l'un des groupes $-CH_2-CH=$, $-CH_2-CH_2-CH_2-$, $-CH_2-CH_2-CH=$ ou $-CH=CH-CH=$ ou un groupe dérivé de l'un de ceux-ci dans lequel un ou deux atomes d'hydrogène sont remplacés par des groupes éther.

3. Procédé selon la revendication 2, caractérisé en ce que l'on convertit un composé de formule I dans laquelle $A^1$ représente l'un des groupes $-CHR-CR'=$, $-CH_2-CR=$, $-CH_2-CH_2-CH_2-$, $-CH_2-CHR-CH_2-$, $-CH_2-CRR'-CH_2-$, $-CH_2-CH_2-CR=$, $-CH_2-CHR-CR'=$ ou $-CH_2-CRR'-CH=$ et R et R' représentent des groupes éther.

4. Procédé selon l'une des revendications 1 à 3, caractérisé en ce que l'on convertit un composé de formule I dans laquelle les groupes éther éventuellement présents dans $A^1$ sont des groupes méthoxy, isobutyloxy ou 1-méthoxy-1-méthyloxy ou bien encore deux groupes éther géminés forment ensemble un groupe éthylène-dioxy ou bien deux groupes éther vicinaux forment ensemble le groupe acétonide, ou bien encore, dans le cas d'alpha-hydroxycétones énolisées protégées, les groupes éther vicinaux forment un dimère par éthérification.

5. Procédé selon l'une des revendications 1 à 4, caractérisé en ce que l'on fait réagir avec un composé de formule II dans laquelle les groupes éther eventuellement présents dans $R^1$ sont des groupes méthoxy isobutyloxy ou 1-méthoxy-1-méthyléthoxy, ou bien deux groupes éther forment ensemble un groupe éthylène-dioxy.

6. Procede selon la revendication 5, caractérisé en ce que l'on fait réagir avec le composé de formule II dans laquelle $R^1$ représente le groupe $-CH_2OLi$.

7. Procédé selon l'une des revendications 1 à 6, caractérisé en ce que l'on effectue la réduction à l'aide d'un composé de formule IV dans laquelle Me représente le lithium ou le sodium et x est égal à 2.

8. Procédé selon la revendication 7, caractérise en ce que l'on effectue la réduction à l'aide de l'hydrure de sodium et de bis-(2-méthoxyéthoxy)-aluminium.

9. Procédé selon l'une des revendications 1 à 8, caractérisé en ce que l'on effectue la réaction dans un éther ou un hydrocarbure saturé ou aromatique.

10. Procédé selon la revendication 9, caractérisé en ce que l'on effectue la réaction dans le tétrahydrofuranne.